# EUROPEAN PATENT APPLICATION

(11) **EP 1 510 585 A1**
(43) Date of publication of application: **02.03.2005**
(21) Application number: 04450028.8
(22) Date of filing: 13.02.2004
(51) Int. Cl.: C12N 15/82, C12N 9/10

(54) **A method for regulating plant growth**

(30) Priority: 01.09.2003 EP 03450194
(71) Applicant: Universität für Bodenkultur Wien, 1180 Wien (AT)
(72) Inventor: Poppenberger, Brigitte, 2362 Biedermannsdorf (AT); Adam, Gerhard, 1160 Vienna (AT); Luschnig, Christian, 1170 Vienna (AT); Glössl, Josef, 1140 Vienna (AT)
(74) Representative: Sonn & Partner Patentanwälte

(57) **Abstract**

Described is a method for regulating plant growth which is characterised in that the activity of a brassinosteroid specific glycosyltransferase is influenced.

## Description

The invention relates to a method for regulating plant growth.

The regulation of plant growth has been a crucial driving force for many developments in plant physiology, especially in molecular biology assisted plant development.

Plant growth is accomplished by orderly cell division and tightly regulated cell expansion. In plants the contribution of cell expansion to growth is of much greater significance than in most other organisms. All plant organs owe their final size to a period of significant cell elongation which usually follows active cell division. Cell elongation is a major factor in growth. Coordinate control of plant growth is regulated by both external stimuli and internal mechanism. Of the external signals the most obvious is light. The internal components of plant signalling are generally mediated by chemical growth regulators. Thus, plant growth in response to environmental factors is modulated by plant hormones acting alone or in concert and growth depends on regulated cellular events, such as division, elongation and differentiation.

Gibberellic acid (GA) and cytokinins promote flowering; in addition, GA stimulates stem elongation, whereas cytokinins have the opposite effect, reducing apical dominance by stimulating increased axillary shoot formation. Conversely, auxins promote apical dominance and stimulate elongation by a process postulated to require acidification of the cell wall by a K⁺-dependent H⁺- pumping ATPase. In addition to the classic hormones, such as auxin and GA, brassinosteroids (BRs) have been discovered to be important in growth promotion. Brassinosteroids are plant hormones with a polyoxygenated steroid structure showing pronounced plant growth regulatory activity (see reviews of Zullo et al. (Braz.J.Plant Physiol., 14(3) (2002), 143-181); Bishop et al., The Plant Cell, S97-S110 (Supplement 2002) (2002)). These brassinosteroids show structural similarity to the steroid hormones of vertebrates and insects. Plant mutants defective in brassinosteroid biosynthesis or perception exhibit dwarfism and reduced fertility, and reveal the need for brassinosteroids during growth and development. For example, brassinosteroid signalling in Arabidopsis thaliana and Oryza sativa - dicotyledonous and monoctotyledonous models, respectively - is mediated by the receptor kinases BRI1 and OsBRI1. The extracellular domain of BRI1 perceives brassinosteroids and the signal is mediated via an intracellular kinase domain that autophosphorylates Ser and Thr residues and apparently has the potential to phosphorylate other downstream signalling components. BRI1 transduces steroid signals across the plasma membrane and mediates regulatory effects on gene expression (Müssig et al., TRENDS in Endocrinology & Metabolism 12(9) (2001), 398-402).

Plants with short stature are advantageous in many agriculturual settings, as the harvesting index is improved (more grain, less straw) and the plants are often more resistant to lodging due to rain, wind and planting in high density. Most of the progress of the so called green revolution has been achieved by altering plant hormone homeostasis. Elongation of plants is mediated by gibberellins, auxins and brassinosteroids. Several molecular mechanisms have been elucidated which cause phenotypes of agricultural value.

Traditionally, the control of plant stature has been through the process of selective breeding. Often dwarf plants are chosen for their ornamental value or their improved ability to survive under mechanical stress, such as high wind. However, this breeding process can take many years. An alternative way to rapidly create dwarf plants is by the exogenous application of certain organic compounds, such as the gibberellin biosynthesis inhibitor uniconazole. However, these compounds are expensive and must be applied throughout the plant life cycle.

Alteration of gibberellin levels or perception has also been shown to effectively achieve a dwarfing process in rice. Mutant (loss of function) alleles of gibberellic acid biosynthetic gene have been shown to be responsible for sdl dwarf rice (Sasaki et al., Nature 416 (6882) (2002), 701-702). Dwarfing in rice can also be caused by overexpression/gain of function of a GA catabolic gene or mutations in transcription factors acting as growth repressors that are normally suppressed by GA (e.g. wheat reduced height Rht genes).

Dwarfing in maize and sorghum has been achieved by loss of function alleles of an auxin transport protein (ABC transporter of the PGP type; Multani et al., Science 302 (5642) (2003), 81-84)).

In rice and barley two types of dwarfing genes have been characterised: By a loss of function allele of a cytochrome P450 (Hong et al., Plant Cell 15(12) (2003), 2900-10). gene involved in brassinosteroid biosynthesis and by a missense mutation in a brassinosteroid receptor gene homologous to Arabidopsis. In rice, a similar dwarfing is most likely caused by a defect of the corresponding gene (Chono et al., Plant Physiol. 133(3) (2003), 1209-1219).

Therefore, up to date the predominant novel ways of achieving growth control, especially dwarfing (however, in certain constellations also the other direction (elongation) is desired) has been reached by either mutations in plant hormone biosynthesis or receptor genes or by exogenous application of these plant hormones or inhibitors thereof.

It is an object of the present invention to provide alternative methods for influencing plant growth, i.e. by specifically regulating the plant growth into the desired direction.

The present invention therefore provides a method for regulating plant growth which is characterised in that the activity of a brassinosteroid specific glycosyltransferase is influenced.

Brassinosteroid specific glycosyltransferases inactivate a brassinosteroid plant hormone by attachment of a sugar residue. The selective control of the activity of these brassinosteroid specific glycosyltransferases is therefore an elegant means for regulating plant growth: Enhancing the activity of such an enzyme inactivates the plant brassinosteroid hormones and may therefore lead to dwarfing, an inhibition of the (physiological) brassinosteroid specific glycosyltransferase activity leads to enhanced action of these plant hormones, improved growth.

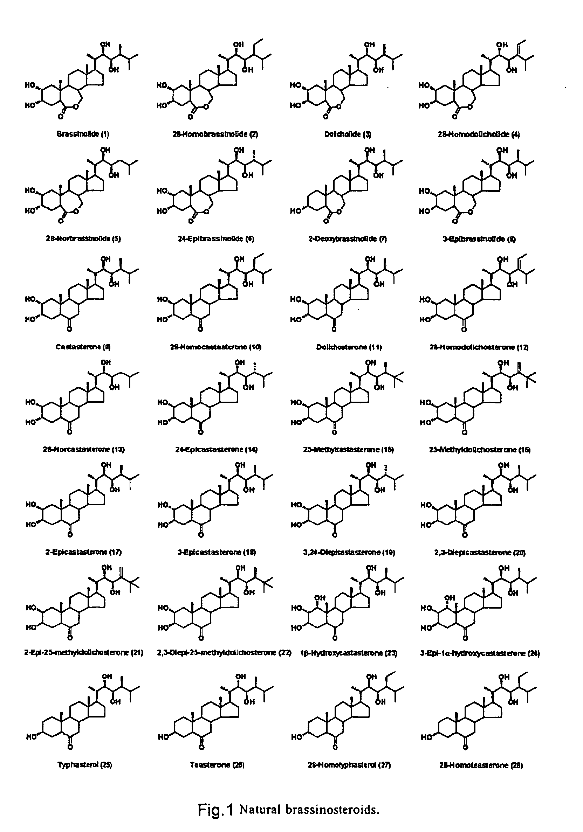

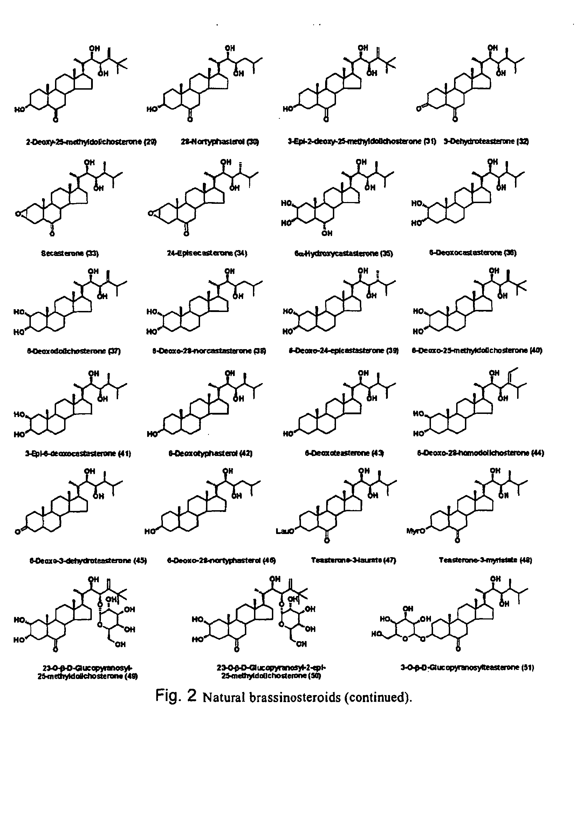

The glycosyltransferases to be used according to the present invention have a specific glycosyltransferase activity to the naturally occurring brassinosteroids, as e.g. given in Figs. 1 and 2, wherein the general structural formula of natural brassinosteroids are given.

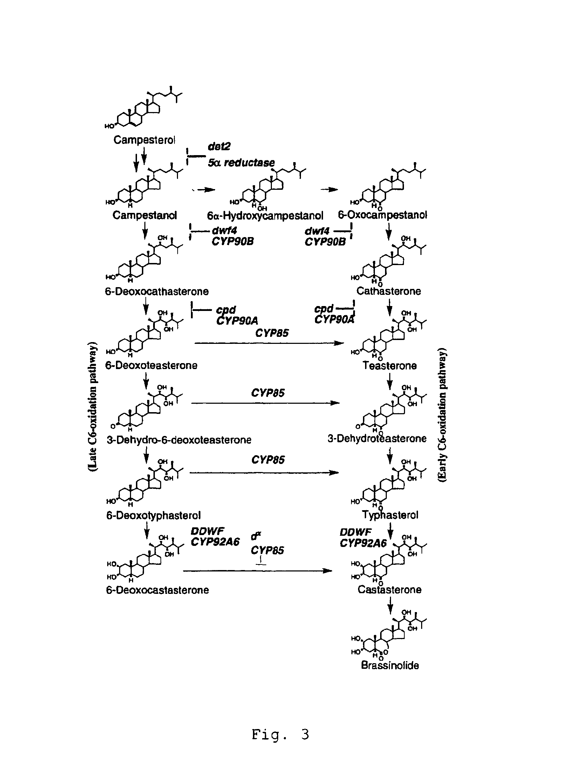

The eludication of brassinosteroid biosynthesis and metabolism was important for determining their biologically active forms and for understanding how their endogenous levels are regulated to promote adequate plant growth and development. It was verified that brassinolide biosynthesis begins with the reduction of Campesterol to Campestanol which is oxidised to 6α-Hydroxy-campestanol and this to 6-Oxocampestanol. There are, however, 2 routes of biosynthesis leading to Castasterone which is then transformed to Brassinolide (BL). These 2 routes are called the late C-6 oxidation pathway and the early C-6 oxidation pathway (see Fig. 3). In principle, action of glycosyltransferases on these brassinosteroids can be made in any step during biosynthesis and action of these hormones. It is, however, preferred to act on the common compounds of both biosynthetic pathways, e.g. on Campesterol or Campestanol on the one hand or on Castasterone or Brassinolide on the other hand. Alternatively, for promoting either the late C-6 oxidation pathway or the early C-6 oxidation pathway, specific glycosylation of these intermediate products leads to pathway specific regulation of the hormone synthesis.

A brassinosteroid specific glycosyltransferase has preferably the brassinoid specific glycosylation as its main activity (under physiological conditions in the plant). Especially in view of the fact that many glycosyltransferases have further (side) activities, also side specificities may be used according to the present invention, yet less preferred.

According to a preferred embodiment of the present invention plant growth is reduced by enhancing the expression of the brassinosteroid specific glycosyltransferase. This results in glycosylation of the brassinosteroid and therefore reduction of its biological activity. Enhancing the activity of the glycosyltransferase may preferably be achieved by exogenous supplementation of transferase activators, by addition of inhibitors to competing substances, especially enzymes, or by raising the amount of active glycosyltransferase molecules in the plant. Preferably, this can be achieved by plant molecular biology: typically a heterologous UGT with substrates specificity for brassinosteroids is placed downstream of a strong constitutive or inducible, preferentially tissue specific promoter. Also the expression of an endogenous UGT can be altered by site specific introduction of a heterologous or endogenous strong promoter by homologous recombination, at least in rice (e.g. Terada et al., Nat.Biotech. 20 (10) (2003); 1030-1034). "Heterologous" means that the glycosyltransferase or expression regulating element is a gene or element not naturally present in this location (a gene or sequence being naturally present is referred to as an "endogeneous" glycosyltransferase or in the genome sequence) in the wild type plant, e.g. an expression regulating element from another plant species or from another locus of the same species is inserted or that a glycosyltransferase from a different plant species is introduced.

Ways of inhibiting a given glycosyltransferase activity in a specific plant or plant cell include knock out mutations of the genes or essential parts thereof or of regulating elements needed for expression, applying antisense technology or applying RNA interference (RNAi)technology (see e.g. Nature Reviews "RNAi collection" (December 2003), 1-43).

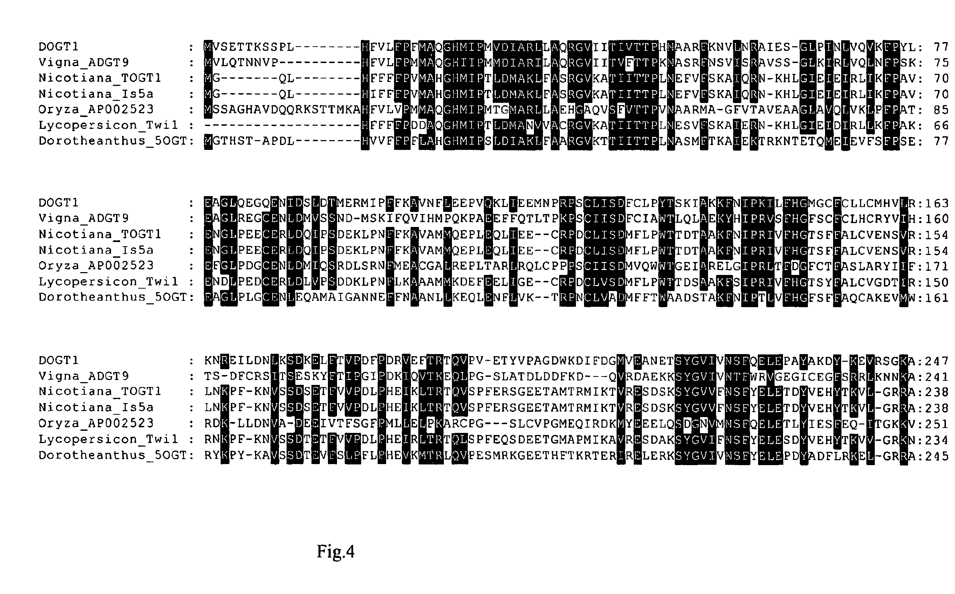

In principle, any brassinosteroid specific glycosyltransferase may be used according to the present invention. It is, however, preferred to use a glucosyltransferase since brassinosteroids have proved to be most susceptible to glucosylation. Preferred examples of glucosyltransferases to be used according to the present invention are UDP-glucosyltransferases, preferably those corresponding to sub-family 73C of Arabidopsis thaliana, especially UDP-glucosyltransferases 73C5, 73C6 and 73C4. Amino acid sequence of UDP-glucosyltransferase 73C5 is given in Fig. 4.

Of course, also homologues to these glucosyltransferases from other plants are also preferred. Specifically preferred therefore are all UDP-glucosyltransferases corresponding to sub-family 73C4 and 73C5 of Arabidopsis from other plants, especially those having a high amino acid amino acid identity to the polypeptides of Arabidopsis, i.e. a sequence identity being higher than 70%, preferably higher than 80%, especially higher than 90% identity, especially in the N-terminal substrate specificity domain. Sequence identities may e.g. be calculated according to Dayhoff ("Atlas of Protein Sequences and Structures" 5 Suppl.3: 353-358, NBRF, Washington, USA) or Gribskov (NAR 14(6) (1986), 6745-6763). Alternatively, amino acid identity may also be calculated for a given region (e.g. having a length of at least 100 amino acid residues) of the enzyme comprising the enzymatically important region.

In the present method for regulating plant growth a preferred method comprises introducing a tissue specific promoter for the brassinosteroid specific glycosyltransferase, especially a stem specific promoter into a plant. By choosing organ specific promoters it is possible to direct the dwarfing or elongation to specific target tissue. For example in producing dwarfing an active and high expression promoter acting specifically in the cells of the stem may be provided which is not active in leave or reproductive organs. This technique is in principle known to the skilled man in the art and may be applied to the present invention with the knowledge given in the present description (an approach based on expression of a GA catabolic enzyme GA2-oxidase is published in Sakamoto et al., Nat.Biotech. 21(8) (2003), 909-913).

Preferably, the present method is applied to plants selected from the group containing Arabidopsis, tobaccco (as model plants), and important crop plants (ranging from stapel food plants such as rice, maize, wheat, barley, sorghum to vegetables and to fruit trees. Another important group of plants that can be altered in shape according to the present invention are forest trees, and ornamental plants (flowering plants, bonsai shrubs etc). These plants are specifically preferred due to their physiology and/or importance to food industry.

As mentioned above, preferred brassinosteroid specific glycosyltransferases are glucosyltransferases, especially glucosyltransferases being specific for Campesterol, Campestanol, Brassinolide, Stigmasterol, teasterone, methyldolichosterone, epibrassinolide and epicastasterone.

Preferably, the plant growth may be reduced by the glycosylation, especially the glucosylation of the C₂-OH, C₃-OH, C₂₃-OH, C₂₅-OH, C₂₆-OH and/or C₂₇-OH of brassinosteroids by brassinosteroid specific glycosyltransferases because these positions (especially C₂, C₃ and C₂₃) have been shown to be specifically preferred for glucosylation in (24-epi-)brassinosteroids (see Zullo et al. (2002)). Indeed, brassinosteroid specific glycosyltransferases often show high specificity as different enzymes catalyse different transformations. Moreover, these enzymes can be located (physiologically) in different plant organs. Therefore, not only a single receptor site for a brassinosteroid is present, but there are different receptor sites in different enzymes in which different brassinosteroid molecules are able to exhibit one of the many brassinosteroid physiological acitivities. Each receptor site must need different structural requirements for exhibiting the maximal activity which makes glycosylation, especially glucosylation of brassinosteroids an effective means for regulating brassinosteroid activity inside plant cells.

Preferably, the method according to the present invention comprises the introduction of a inducible promoter for the brassinosteroid specific glycosyltransferase, preferably a tissue specific promoter, especially a stem specific promoter into a plant.

The present invention makes use of a completely different concept of influencing plant growth, especially with respect to enabling dwarfism, compared to the prior art.

The mode of action is not dependent on gibberellin or auxin.

WO 97/35986 A1 discloses that mutation of the gene for a cytochrome P450-type hydroxylase, an enzyme acting in BR biosynthesis pathway, can induce dwarfism, if the activity of this gene product is reduced or eliminated.

A similar approach was taken in WO 00/47715 A2 wherein "dwf 4 polynucleotides" are used for generating dwarf plants (dwf 4 also encodes for a cytochrome P450 enzyme that mediates multiple steps in synthesis of brassinosteroids).

Also in US 2002/0073446 A1 a cytochrome P450 is used for generating reduced stature when expressed at increased levels. The enzyme described in this US-A1 catalyses the C₂₆-hydroxylation of brassinosteroids, more specifically C₂₆-hydroxylation of ecdysone, thus preventing predators from undergoing melting after feeding on plants expressing high levels of the cytochrome P450.

In EP 1 275 719 A1 rice dwarf mutant d61 is described. This was shown to be dependent on the suppression of the OsBRI1 gene, a receptor-like kinase. Later it was confirmed that binding of brassinolide (BL), either directly or through an accessary factor (e.g. a steroid binding protein), to the leucin-rich repeat domain of (Os)BRI1 results in autophosphorylation of BRI1 (see: Bishop et al. (2002)).

EP 1 209 227 A2 also discloses a nucleic acid molecule encoding a dark-inducible cytochrome P450 hydroxylase that catalyses the brassinosteroid biosynthesis through C-2 hydroxylations in plants. This gene was used for regulating plant growth (dwarfism and elongation).

US 2003/0199684 A1 discloses a plant gene capable of controlling a signal transduction system for brassinosteroid hormone. This should enable growth promotion, yield increase and quality improvement by selectively manipulating the brassinosteroid signal transduction.

All these methods according to the prior art using the brassinosteroid hormone action relied upon either controlling and manipulating the brassinosteroid biosynthesis or by controlling and influencing brassinossteroid signal transduction molecules, such as receptors. In contrast to these approaches according to the prior art, the present invention directly aims at the brassinosteroid hormone molecule by inactivating the hormone molecule by in vivo glycosylation (e.g. for enabling dwarfism) or - alternatively - by reducing or eliminating this glycosylation activity.

According to another aspect, the present invention relates to transgenic plants or a transgenic (plant) cell containing recombinant glucosyltransferase and/or a recombinant expression regulating element, especially a promoter region for a glucosyltransferase. These plants or (plant) cells may be used in a method according to the present invention.

The present invention also relates a genetically modified cell or organism, especially a plant or plant cell comprising a non-naturally occurring (transgenic) glucosyltransferase in its genome with growth properties being different from the wild type, especially characterised that it shows a "dwarf" phenotype due to the presence of such a glucosyltransferase as transgene. Alternatively, an endogenous glucosyltransferase in a cell may be subjected to a different transgenic expression regulating region or element, such as a promoter leading to an enhanced expression of the endogenous or exogenous glucosyltransferase, i.e. an enhanced expression activity of a homologous (or heterologous) glucosyltransferase due to transgenic promoters. Although such cells or plants may show a "dwarf" phenotype, if properly adjusted to the cell and switched-off (if the promoter to be used is a switchable promoter), such plant cells and plants can be produced by applying standard methods to the teachings of the present invention. Such "dwarf" plants are a specifically preferred embodiment of the present invention.

Similarily, transformation of plants with other heterologous brassinosteroid specific glycosyltransferases, such as fucosyl-transferases or xylosyltransferases or especially rhamnosyl-transferase may be used to construe specific dwarf plants. However, use of glucosyltransferases is preferred due to the fact that (UDP-) glucose is the physiological group for substituting especially the -OH groups of brassinosteroids.

According to another aspect the present invention enables a method for producing glucosylated brassinosteroids wherein a brassinosteroid is contacted with a glucosyltransferase in the presence of an activated glucose (e.g. UDP-glucose). Accordingly, the above mentioned enzyme specificities can be properly used for producing specifically glucosylated brassinosteroid derivatives. If a brassinosteroid is glucosylated in a non-enzymatic (chemical) way, a crude mixture of glucosylation products is obtained (each free hydroxygroup is in principle a preferred acceptor side for glucosyl residues). Acetylation as protection means for such group has also its drawbacks with respect to specificity (also all hydroxygroups are acetylated). In using substrate and side specificity of a given glucosyltransferase, the present invention provides a method for producing well-defined glucosylated derivatives of brassinosteroids. If more than one hydroxygroup is glucosylated or if more than one position is capable of being glucosylated by a given glucosyltransferase, such a limited number of products, e.g. 2 to 5, may easily be separated by suitable separation means, such as HPLC.

For this method, not only the natural brassinosteroids (as depicted in Figs. 1 and 2) can be used for glucosylation, but also other brassinosteroids, e.g. brassinosteroid derivatives and analogues, preferably those depicted in Figs. 5, 6, 21-26 and 129 in Zullo et al. (2002) or the metabolised forms of Figs. 12-20 in Zullo et al. (2002).

Since uridine diphosphate glucose (UDP-glucose, e.g. glucose which is produced by enzymes, such as pyrophosphorylases) is a preferred activated biological form of glucose, a preferred embodiment of the present method uses UDP-glucosyltransferase as glucosyltransferase and an UDP-glucose as activated glucose (=cosubstrate), however, any form glucose, which can be utilised by a glucosyltransferase (e.g. ADP-glucose, CDP-glucose, or any other form of biologically and synthetically activated glucose being suitable substrates for transfer to brassinosteroids by glucosyltransferases) can be used according to the present invention.

A glucosyltranferase according to the present invention is defined as a (naturally) occuring or synthetically (recombinantly) designed or produced enzyme which is capable (as its main or as a side activity) of transferring a glucose moiety to a substrate molecule using an activated glucose as co-substrate thereby producing a glucosylated substrate molecule.

The mode of action of the present invention is not dependent of gibberellin, the inactivation or enhanced activation of a brassinosteroid molecule by means of brassinosteroid specific glycosyltransferases.

The present invention is further illustrated by the following examples and figures, yet without being restricted thereto.

Fig. 1 and 2 show formulae of natural brassinosteroids.

Fig. 3 shows biosynthesis of brassinolide via the late C-6 oxidation pathway and via the early C-6 oxidation pathway; this figure shows a simplified scheme of the BR biosynthesis pathway including steps of early and late C-6 oxidation that lead to the synthesis of BL (Noguchi et al., 2000, Plant Physiol. 124:201-209; Shimada et al., 2001, Plant Physiol. 126: 770-779). Mutations in genes coding for key biosynthetic enzymes are in bold det-2, DEETIOLATED-2, steroid 5α reductase (Li et al., 1996, Science 272:398-401), dwf4, DWARF4 cytochrome P450 CYP90B, C-22 hydroxylase (Choe et al., 1998, Plant Cell 10: 231-243); cpd, CONSTITUTIVE PHOTOMORPHOGENIC and DWARFISM cytochrome P450 CYP90A, C-23 hydroxylase (Szekeres et al., 1996, Cell 85: 171-182), d^{x} DWARF cytochrome P450 CYP85, C-6 oxidase (Bishop et al., 1996, Plant Cell 8: 959-969; 1999, PNAS 96: 1761-1766; Shimada et al., 2001); DDWF, DARK-INDUCED dwf-LIKE-1, cytochrome P450 CYP92A6, C-2 hydroxylase (Kang et al., 2001, Cell 105:625-636).

Fig. 4 shows amino acid alignment of plant UDP-glucosyltransferases with high amino acid similarity to DOGT1 (= Arabidopsis thaliana UGT73C5). The regions implicated (Vegt et al., Plant J. 19 (1999), 507-519) in acceptor substrate binding (dotted) and the UGT consensus sequence motif (dashed) are indicated by boxes below the sequences. The triangle above the sequence in the hypothetical acceptor binding region marks the lysine 136 in DOGT1 which has been altered by in vitro mutagenesis. The genbank accession numbers of the predicted proteins are: ADGT-9, glucosyl-transferase-9 of Vigna angularis (AB070752); DOGT1, Phenylpropanoid:glucosyltransferase 1 of Nicotiana tabacum (AF346431); IS5a of Nicotiana tabacum (U32644); putative glucosyltransferase of Oryza sativa (AP002523); Twi1 of Lycopersicon esculentum (X85138); Betanidin-5-O-glucosyltransferase of Dorothenathus bellidiformis (Y18871).

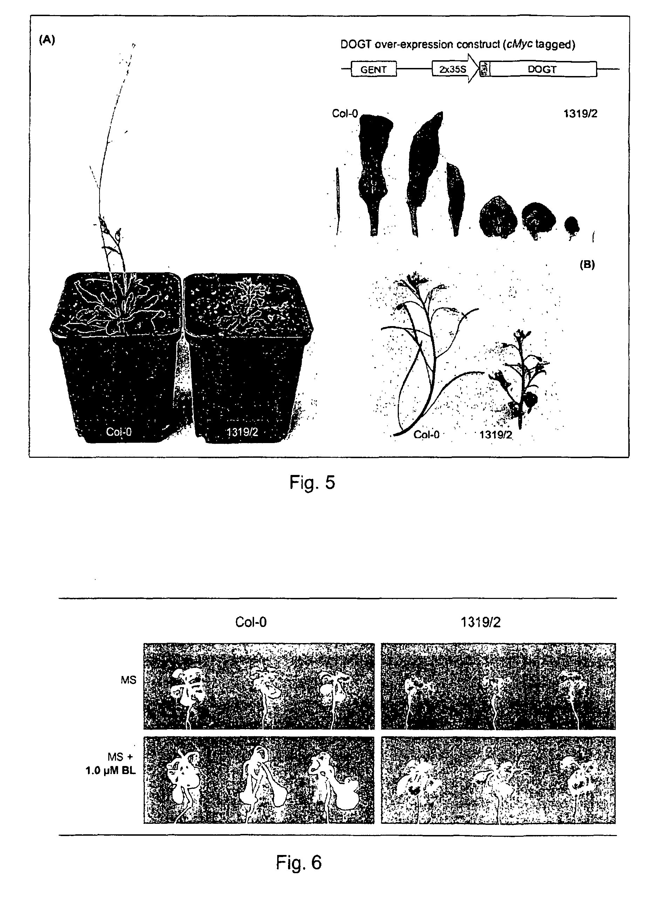

Fig. 5 shows homozygous A. thaliana constitutively expressing high amounts of DOGT1T exhibit a brassinosteroid-deficient phenotype apparent in a dwarf stature, dark green, small, round, wrinkly leaves with short petioles, very short siliques, reduced fertility, reduced apical dominance with an increased number of influorescenses, which leads to a bushy appearance and delayed senescence (A). 5 week old light grown plants, left wild-type Col-0, right 1319/2, a line with high DOGT1 expression (B). Phenotype of Col-0 and 1319/2 leaves, flowers and siliques after 5 weeks of growth.

Fig. 6 shows the BR-deficient phenotype can be restored to wild-type by externally applied BL. 10 day old seedlings were transferred to MS media resp. MS media containing 1.0 µM BL and incubated for another 10 days before phenotypic analysis. Left: Wild-type exhibits the typical growth response to externally applied BL, which includes cell expansion of young aerial tissues, leaf bending and chlorosis. Right: Seedlings of lines expressing DOGT1 grown on MS media show a BR-deficient phenotype (short hypocotyls and smaller leaves with shorter petioles compared to wild-type). Externally applied BL restores wild-type growth in DOGT1 lines.

### EXAMPLES

For illustrating the present invention, in the examples hereinafter the cloning of an Arabidopsis UGT by functional expression in yeast and its characterization is described. Constitutively enzyme leads to a dwarf phenotype. The methods used in these examples are also directly applicable to other glycosyltransferases, especially glucosyltransferases without undue experimental burden.

### EXPERIMENTAL PROCEDURES

**Yeast strains.** The yeast strains used in this work are derived from YPH499 (Mat a, ade2-101oc, his3-Δ200, leu2-Δ 1, lys2-801a, trp1- Δ 1, ura3-52) (Sikorski et al., Genetics 122 (1989), 19-27). The relevant genotype of YZGA452 is pdr5Δ::TRP1, pdr10Δ::hisG, snq2Δ::hisG, yor1Δ::hisG. YZGA515 (pdr5Δ::TRP1, pdr10Δ::hisG, pdr15Δ::loxP-KanMX-loxP, ayt1Δ::URA3) was constructed by disruption of the acetyltransferase AYT1 in strain YHW10515.

**Plant material and growth conditions.** A. thaliana experiments were conducted with the wild-type ecotype Columbia-0 (Col-0). For propagation seeds were sterilized, plated on standard MS growth medium (Murashige et al., Plant Physiology 15 (1962), 473-497) supplemented with 1.0 % sucrose and 1.0% phytagar (Life Technologies) and subjected to a 2 day dark treatment at 4°C to synchronize germination. The seedlings were grown for 2 weeks in a controlled environment of 16h/8h light-dark cycle (140 µmol m⁻² sec⁻¹ white light) at 22°C before they were transferred to soil and grown at 20°C and 55% humidity under continuous white light.

**Arabidopsis thaliana cDNA library screen in yeast.** The ATP-binding cassette (ABC) transporter deficient Saccharomyces cerevisiae strain YZGA452, which is hypersensitive to DON, was transformed with an A. thaliana cDNA library constitutively expressed under the control of the phoshoglucerate kinase (PGK1) promoter (Minet et al., Plant J. 2 (1992), 417-422). A total of 10⁷ transformants were selected on minimal medium lacking uracil and transferred to media containing 180 ppm DON, sufficient to completely inhibit growth of yeast transformed with the empty library plasmid. Colonies that showed resistance were isolated and from candidates that formed single colonies on toxin containing media, the plasmid dependency of the phenotype was tested by plasmid DNA preparation and retransformation of YZGA452. The NotI fragment containing the cDNA insert of the candidate (which was named DON glucosyltransferase 1, DOGT1) was subcloned into pBluescript SKII+ (Stratagene) and sequenced.

**Constitutive expression and immunodetection of the DON-glucosyl** **transferase (DOGT1) and close homologues in yeast.** The intronless open reading frames (ORFs) of DOGT1 (UGT73C5, locus At2g36800) and 5 of its closest homologues (UGT73C1, At2g36750; UGT73C2, At2g36760; UGT73C3, At2g36780; UGT73C4, At2g36770; UGT73C6, At2g36790) were PCR amplified (Triple Master PCR System, Eppendorf) from genomic DNA using gene specific primers containing flanking HindIII and NotI restriction sites at the 5' and 3' ends, respectively
(DOGT1, fw: 5'-ACTAAGCTTGGAATCATGGTTTCCGAAACA-3', rv: 5'-AAGCGGCCGCATACTCAATTATTGG-3'; 73C1, fw: 5'-CTAAGCTTGGAAT-CATGGCATCGGAATTTCG-3', rv: 5'-TAGCGGCCGCATTCATTTCTTGGGTTGTTC-3';
73C2, fw: 5'-CTAAGCTTGGAATCATGGCTTTCGAGAAGACC-3', rv: 5'-TAGCG-GCCGCATTCAACTCTTGGATTCTAC-3';
73C3, fw: 5'-CTAAGCTTGGAATCATGGCTACGGAAAAAACC-3', rv: 5'-TAGCG-GCCGCATTCATTCTTGAATTGTGC-3';
73C4, fw: 5'-CTAAGCTTGGAATCATGGCTTCCGAAAAATC-3', rv: 5'-TAGCGGC-CGCATTCAGTTCTTGGATTTCA-3';
73C6: 5'-CTAAGCTTGGAACATGTGTTCTCATGATCCT-3', rv: 5'-TAGCGGCCGC-ATTCAATTATTGGACTGTGC-3'). The PCR products were cloned into the HindIII + NotI cloning sites of the yeast expression vector pYAK7 (PADH1-c-Myc-PDR5 LEU2 2µ), replacing the PDR5 gene. The vector pYAK7 was constructed by first inserting the double stranded linker 5'-GGATGCCCGAACAAAAGTTAATTTCAGAAGAGGACTTAT-CAAAGCTTGAGGCCTCGCGA into the SmaI site of vector pAD4Δ (Ballester et al., Cell 59 (1989), 681-686), thereby generating the N-terminal c-Myc epitope and a HindIII site, into which a genomic HindIII fragment containing the yeast PDR5 was inserted in frame.

The tagged UGT constructs were verified by sequencing and used to transform the yeast strain YZGA515. The empty vector (HindIII + NotI digested and religated pYAK7) was used as a control. Transformants were selected on minimal media lacking leucine.

For immunodetection, the extraction of proteins from yeast cells was performed as described by Egner et al. (Mol.Cell.Biol. 15 (1995), 5879-5887). Western blot analysis was conducted with a primary mouse anti c-Myc antibody (1:5000, clone 9E10, Invitrogen).

**Domain shuffling.** The ORFs including the N-terminal c-Myc tag of DOGT1 and its closest homologue UGT73C6 were isolated from the yeast expression vectors by SmaI + NotI digestion, cloned into vector pBluescript SKII+ (which was cut with XhoI and treated with Klenow enzyme, and subsequently digested with NotI). The resulting plasmids were digested with HindIII and a conserved EcoRI site present in both genes that cleaves DOGT1 at nucleotide position 565 (73C6 at 568). Hybrids were constructed by ligation of the N-terminal part of one gene to the C-terminal part of the other. The resulting genes were moved back into the yeast expression vector pYAK7 using the HindIII and NotI restriction sites. Yeast strain YZGA515 was used as a host to test the constructs for altered detoxification abilities.

**Site directed mutagenesis.** Mutations were constructed by overlap extension PCR (Pagulis et al., Meth.Mol.Biol. 57 (1996), 167-176) using overlapping mutant primers DOGT1-K136E-fw (5'-TACAAGCGAAATCGCCAAGAAGTTCA-3') and DOGT1-K136E-rv (5'-CTTCT-TGGCGATTTCGCTTGTATAAG-3') and flanking primers DOGT1IpYAK7-fw-a (5'-ACTAAGCTTGGAATCATGGTTTCCGAAACA-3') and DOGT1-EcoRI-rv (5'-TCTTGTGAATTCAACTCTATC AGGA-3') for mutagenesis of DOGT1, and mutant primers 73C6-E137K-fw (5'-TACAAGCAAAATCGCC AAGAAGTTCAA-3') and 73C6-E137K-rv (5'-ACTTCTTGGCGATTTTGCTTGTATT-3') and flanking primers 73C6pYAK7-fw (5'-TAAGCTTGGAATCATGTGTTCTCATG-ATCCT-3') and DOGT1-EcoRI-rv for 73C6 mutagenesis. The resulting PCR products were cloned as HindIII+EcoRI fragments into the corresponding genes present in vector pBluescript SKII+. After sequencing, the ORFs were moved as HindIII + NotI fragments back into the yeast expression vector pYAK7 (replacing the PDR5 gene) and the resulting plasmids were transformed into strain YZGA515 to analyze the detoxification properties of the engineered UGTs.

**Heterologous expression of DOGT1 in Escherichia coli.** The DOGT1 protein was expressed in Escherichia coli XL1-blue as a GST fusion. The DOGT1 gene was released from the yeast expression vector by HindIII digestion and Klenow fill in, followed by a NotI digest. The resulting fragment was cloned into the SmaI + NotI sites of the GST gene fusion vector pGEX-4T-3 (Amersham Pharmacia). Recombinant fusion protein was purified using glutathione-coupled Sepharose (Amersham Pharmacia) according to the manufacturer's instructions.

To test the effect of the N-terminal GST tag on activity, the gene encoding the fusion protein was PCR amplified using DNA polymerase with proof reading activity (Pfu polymerase, MBI) and the fusion protein specific primers GSTDOGT1pYAK7-fw (5'-TCAC-CCGGGAAACAGTAATCATGTCC-3') and GSTDOGT1pYAK7-rv (5'-CGAGGCAG-ATCGTCAGTCAGTC-3'). The PCR product was cloned HindIII+NotI into the yeast expression vector pYAK7.

**Enzyme assays.** The glucosyltransferase activity assay mix contained 1 µg of recombinant GST-fusion protein, 10 mM 2-mercaptoethanol, 50 mM Tris/HCl pH 7.0, 0.5 mM radioactive labeled UDP-[¹⁴C] glucose (4.4*10³ cpm, NEN Life Science Products, USA), 0.01% BSA and 1 mM of acceptor substrate (dissolved in DMSO in 20 mM stock solutions). The reactions were carried out in volumes of 20 µl at 30°C for 1 h, stopped by adding 2 µl trichloroacetic acid (240 mg/ml), frozen and stored at -20°C. Analysis of reaction products was performed by TLC.

**Plant treatment with different stress response related compounds for expression analysis.** For Reverse Transcription (RT)-PCR analysis of mRNA expression of DOGT1 following treatments with DON, salicylic acid (SA), jasmonic acid (JA) and 1-aminocyclopropylcarbonic acid (ACC) seedlings were grown for 2 weeks on vertical MS plates (0.8% phytagar) before they were transferred to liquid MS media. The plants were incubated for 48 h on an orbital shaker (50 rpm) before adding 5 ppm DON, 200 µM SA, 2 µM ACC or 50 µM JA. The compounds were kept in stock solutions dissolved either in 70% ethanol or in DMSO. Treatments with ethanol and DMSO were performed as controls. Plants were harvested at different time points, ground in liquid nitrogen and stored at -70° C until RNA extraction was performed.

**Analysis of mRNA expression of DOGT1 and close homologous by Reverse Transcriptase-PCR.** Total RNA was isolated from plant tissue ground in liquid nitrogen with Trizol Reagent as recommended by the manufacturers (Gibco BRL Life Technologies). RNA was quantified photometrically and visually on a denaturing RNA gel analyzing 5 µg of total RNA. cDNA was synthesized from 1 µg of total RNA (digested with DNa-seI) with 500 ng of a 18-mer oligo(dT) and the reverse transcriptase SuperScript (Gibco BRL Life Technologies). PCR was performed with approximately 2 µl of the 1:20 diluted cDNA using primers that amplify 200 to 400 bp large fragments located in the C-terminal part of the genes to be analyzed (DOGT1RT-fw: 5'-ATCCGGGGTTGAACAGCCT-3', DOGT1RT-rv: 5'-TCAATTATTGGGTTCTGCC-3'; 73C4RT-fw: 5'-GGAGAAAATAGGAGTGTTA-3', 73C4RT-rv: 5'-TCAGTTCTTGGATTTCACT-3'; 73C6RT-fw: 5'- GAGAAACTGGTCGTACAA-3', 73C6RT-rv: 5'-TCAATTATTGGACTGTGCT-3'; UBQ5-U: 5'-GTCCTTCTTTCTG-GTAAACGT-3', UBQ5-D: 5'-AACC CTTGAGGTTGAATCATC-3'). To compare relative amounts of transcripts in the samples, DNA fragments of the UBQ5 were first amplified and normalized sample volumes based on the amount of products corresponding to the UBQ5 transcripts were used for PCR.

**Cloning of plant overexpression constructs and GUS-fusion constructs.** For constitutive overexpression of c-Myc-tagged DOGT1 protein in Arabidopsis, the vector pBP1319 was constructed. It is derived from a modified version of the plant expression vector pPZP221 (Hojdukiewicz et al., Plant Mol.Biol.25 (1994), 989-994). The promoter cassette consisting of two copies of the 35S-promoter and the polyadenylation signal of CaMV strain Cabb B-D was used from the vector p2RT, a modified version of pRT100 (Töpfer et al., NAR 14 (1987), 5890).

The c-Myc-tagged DOGT1-fragment was released by SmaI + NotI digestion (Klenow filled) from the yeast expression vector and cloned into pBluescript SKII+, which was cut with ClaI+SmaI and treated with Klenow enzyme. In the next step, the gene was excised from the resulting plasmid as SalI + BamHI fragment and inserted in the XhoI + BamHI sites of p2RT. The obtained 2x35S c-Myc-DOGT1 cassette was isolated by PstI digestion and cloned into the unique PstI site of pPZP221 after the multiple cloning site in that vector had been destroyed by digesting the plasmid with EcoRI+SalI, filling the sites with Klenow and religating it. In the resulting vector pBP1319, the 2x35S c-Myc-DOGT1 cassette is orientated in the opposite direction than the 2x35S gentamycin resistance marker.

For construction of a transcriptional DOGT1-GUS fusion, the GUS vector pPZP-GUS.1 which originates from pPZP200 and contains the GUS gene from pBI101.1 (inserted HindIII + EcoRI into the MCS), was used (Diener et al., Plant Cell 12 (2000), 853-870). The DOGT1 promoter region was PCR amplified from genomic DNA using DNA polymerase with proof reading activity (Pfu Polymerase, MBI) and specific primers (DOGT1P-GUS-fw: 5'-GTTAAAAGCTTACATGTGCAT-TACGGTCTGTGTGAATA, DOGT1P-GUS-rv: 5'-TTTCGGATCCCATG ATTCAACCT-TAGTAAGAAACTCTC). The resulting product was cloned in frame with the GUS gene HindIII + BamHI into the pPZP-GUS.1 vector. Constructs were confirmed by DNA sequencing.

**Generation and analysis of transgenic A. thaliana.** For all plant transformations, the recA deficient Agrobacterium tumefaciens strain UIA143 (Farraud et al., J.Bact.171 (1989), 5314-5321) which harbors the helper plasmid pMP90 (Koncz et al., Mol.Gen.-Genet.204 (1986), 383-396) was used. A. thaliana was transformed applying the floral dip technique (Clough et al., Plant J. 16 (1998), 735-743). The progeny of 15 independent transformants was selected through three generations to obtain homozygous lines.

For immunodetection of c-Myc-tagged DOGT1, about 200 mg - 500 mg of plant material were homogenized in liquid nitrogen. 300 µl of extraction buffer (200 mM Tris-HCl pH 8.9; 200 mM KCl; 35 mM MgCl₂; 12.5 mM EGTA; 15 mM DTT; 0.6 mM sorbitol) and 15 µl of proteases inhibitor cocktail (Sigma, # 9599) were added to the still frozen samples and the mixture was incubated under vigorous shaking for 15 minutes at 4°C. After centrifugation (14.000 rpm for 15 minutes at 4°C), 200 µl of the supernatants were transferred into fresh tubes and stored at -20°C. Equivalent amounts of protein (50 µg) were used for Western blot analysis which was carried out using primary anti c-Myc antibody purified from hybridoma supernatant (clone 9E10).

For analysis of DON resistance, seeds of homozygous lines exhibiting high DOGT1-expression and Col-0 as control were germinated on MS media containing different concentrations of DON (5 - 30 ppm). Seedlings were grown for 5 weeks before the phenotype was documented. GUS activity was analyzed by staining seedlings or organs of adult plants in X-Gluc solution for 2 to 4 h at 37°C (Jefferson, Plant Mol.Biol.Rep. 5 (1987), 387-405).

### RESULTS

Isolation of the DON-glucosyltransferase (DOGT1) by heterologous expression in yeast. An unbiased functional screen based on heterologous expression of cDNAs in yeast was set up with the goal to identify plant genes that contribute to resistance against mycotoxins. Wild-type Saccharomyces cerevisiae is highly resistant to deoxynivalenol (DON). In order to reduce the amount of toxin necessary for the screen, a strain deficient in four ABC transporters was generated, which are to a large extent responsible for pleiotropic drug resistance in yeast (Wolfger et al., Res.Microbiol.152 (2001), 375-389). Strain YZGA452 (snq2Δ::hisG pdr5Δ::TRP1 pdr10Δ::hisG yor1Δ::hisG) is hypersensitive to a wide range of different xenobiotic substances and natural products, including DON. YZGA452 was transformed with a cDNA expression library of A. thaliana (Minet et al. (1992)), where cDNAs are constitutively expressed under the control of the yeast phosphoglucerate kinase promoter. Ten million transformants were generated and diluted pools of transformants were plated on DON containing medium. After selection of DON resistant yeast colonies and confirmation of plasmid dependency of the phenotype, the insert was subcloned and sequenced.

**DOGT1 is a member of the UDP-glucosyltransferase family of A. thaliana and exhibits high similarity to salicylic acid and wound inducible genes of other species.** The cDNA conferring resistance had a size of 1.75 kb and contained an open reading frame of 1488 bp length encoding a putative uridine diphosphate (UDP)-glucosyltransferase. The identified DON-glucosyltransferase (DOGT1) corresponds to gene UGT73C5 and belongs to sub-family 73C, part of group D of UDP-glucosyltransferases (UGTs) of A. thaliana (33). Arabidopsis UGTs constitute a very large gene family, that has been divided into 14 distinct groups, believed to have originated from common ancestors (Ross et al., Genome Biol. 2 (2001), 3004.1-3004.6). DOGT1 is located in a cluster together with five other members of subfamily 73C on chromosome II (BAC clone F13K3, At2g36800). All six tandemly repeated genes contain no introns, and are highly similar to each other (77 - 89% identity at the amino acid level). The similarity is also very high in the intergenic promoter regions.

A database search with the DOGT1 amino acid sequence revealed high similarity to glucosyltransferases from tobacco (TOGT1, Fraissinet-Tachet et al., FEBS 437 (1998), 319-323; Is5a and Is10a, Horwath et al., Plant Mol.Biol. 31 (1996), 1061-1072) and tomato (Twi-1, Truesdale et al., Plant Physiol. 112, 446), the expression of which has been shown to be elevated following treatment with salicylic acid (SA), fungal elicitors or wounding, and to the betanidin 5-O-glucosyltransferase of Dorotheanthus bellidiformis (Vogt et al. (1999). Two putative, uncharacterized glucosyltransferases from Vigna angularis (ADGT-9) and Oryza sativa with homology to DOGT1 were also included in the amino acid alignment shown in Figure 4. Regions of high similarity were observed in both amino- and carboxy-terminal domains of the deduced amino acid sequences. Indicated in Figure 4 are the hypothetical acceptor substrate binding region (Vogt et al. (1999)) and the UGT consensus sequence (Li et al., JBC 276 (2001), 4338-4343).

**The expression of DOGT1 is developmentally regulated and induced by DON and other stress response related compounds.** To investigate whether the expression of DOGT1 is regulated in a similar fashion as described for the related genes of other plant species, the ORF of the β-glucuronidase reporter gene was placed behind the DOGT1 promoter (P_{DOGT1}-GUS). The tissue specific expression of the transcriptional GUS fusion was examined histochemically in transgenic Arabidopsis homozygous for the fusion gene. The results demonstrated that DOGT1 expression is regulated developmentally and is overall rather low. In seedlings, GUS activity was observed to be root and hypocotyl specific, with the strongest expression in the vascular system, in meristematic tissue of the root tips (in the primary root as well as in lateral roots) and in the vasculature of the hypocotyl right after germination. Staining in the vasculature decreased significantly later in development and a patchy staining pattern appeared in epidermal root cells. In adult plants GUS activity was detected in late stages of flower development in petals and in abscission zones.

Exposure of seedlings to either DON (5 ppm for 4 h) or the ethylene precursor aminocyclopropylcarbonic acid (ACC, 2 µM for 1 h) was found to induce P_{DOGT1}-GUS expression. No induction of expression of the reporter was detected upon SA-treatment (200 µM for 12 h) or treatment with jasmonic acid (JA, 50 µM for 1 h). Semiquantitative reverse transcriptase PCR was applied to validate the results obtained from GUS reporter analyses by detecting changes in mRNA levels of DOGT1, 73C4 and 73C6 following treatment with the same concentrations of DON, SA, ACC and JA as used before.

The results of the reverse transcriptase PCR confirm the DON inducible expression of DOGT1 previously observed with the reporter construct, and furthermore show that also the other two tested UGTs are DON inducible. An increase in the amounts of transcripts was observed already after 1 h of incubation with the toxin, reaching a peak after 4 h and declining again between 6 and 12 h. Interestingly, 73C6 showed a stronger induction of expression by DON than DOGT1 or 73C4 although the data shown below indicate that it does not accept the toxin as a substrate. After SA treatment, DOGT1, 73C4 and 73C6 expression was evident at low levels at 4 h and slightly stronger at 12 h. It has to be noted that the applied concentration of 200 µM SA induced the expression of the 3 genes rather weakly. Jasmonic acid as well as treatment with ACC also lead to weak induction of expression of DOGT1 and UGT73C6 apparent already after 1 h of treatment, but rapidly declining with no transcript accumulation detectable anymore after 2 h of exposure to the compounds.

The high similarity of UGT73C1, C2, C3, C4, C5 (DOGT1) and C6 and their clustered appearance on the chromosome suggests that they have evolved via gene duplication from one ancestral gene and might therefore have related enzymatic properties. To analyze possible similarities in their function, the six ORFs were amplified with specific primers and expressed in yeast as fusion proteins with an N-terminal c-Myc epitope tag. Comparison of transformants expressing tagged or untagged DOGT1 showed that the epitope did not interfere with the DON-protective activity.

The yeast transformants representing the full gene set of the cluster were spotted on media containing increasing concentrations of various trichothecenes. Transformants containing the empty expression vector were used as controls.

### Arabidopsis plants constitutively overexpressing UGT73C5 display a brassinosteroid deficient phenotype and are resistant to externally applied brassinolide

DOGT1-overexpression lines show a typical brassinosteroid-deficient phenotype (Clouse, 2002 Brassinosteroids. In: The Ara*bidopsis* book. American Society of Plant Biologists) that correlates in severeness with the amount of recombinant protein present in the plant. The light grown phenotype exhibited by high-expression lines in soil includes a dwarf stature, darker than wild-type, small, round, wrinkly leaves with short petioles, very short siliques, reduced fertility, reduced apical dominance with an increased number of influorescenses, which leads to a bushy appearance and delayed senescence (Figure 4). The phenotype is already apparent in seedlings grown on agar (Figure 5, upper row, right), which have short hypocotyls and smaller leaves with shorter petioles compared to wild-type.

An over-expression of DOGT1 does not lead to a de-etiolated phenotype when seeds are germinated in the dark, which is a characteristic feature of some BR deficient or BR insensitive mutants like *det2* (Chory *et al.,* 1991, Plant Cell. 3: 445-459), *bin2* (Li *et al.,* 2001a, Plant Physiol. 127:14-22) or *bri1* (Clouse *et al.,* 1996, *Plant Physiol.* 111: 671-678). The hypocotyls are elongated and neither does the apical hock nor do the cotyledons open. Light-regulated cell elongation seems therefore not to be altered in DOGT1-overexpression lines.

Application of brassinolide (the BR with the highest biological activity) restores normal growth in dwarf mutants with defects in BR biosynthesis (Altmann, 1999, Planta.208: 1-11); Clouse and Feldmann, 1999 (Molecular genetics of brassinosteroid action. 163-190. In: Sakurai, A., Yokota, T., Clouse, S.D. (ed.) Brassinosteroids: Steroidal Plant Hormones. Springer, Tokyo, Japan). The effect of externally applied epi-brassinolide (epi-BL) was determined by transferring 10 day old seedlings of wild-type and a high DOGT1-expression line (1319/2) to media containing different concentrations of the brassinosteroid. The plants were incubated for 10 days before the phenotype was analysed. Transgenic plants with high DOGT1 expression were restored to wild-type phenotype, whereas Col-0 exhibited a typical growth response to exogenously applied BL, which includes cell expansion of young aerial tissues, especially of the hypocotyl and leaf petioles, leaf bending, chlorosis and inhibition of root growth (Figure 5).

### DISCUSSION

**Gene evolution and substrate specificity.** DOGT1 is located in a cluster with 5 other members of family 73C on chromosome II (At2g36800) indicative for gene duplication by unequal recombination events. One could speculate that such a gene amplification event provides a selective advantage under toxin stress. DOGT1 and other members in the cluster have very similar protein sequences suggesting redundancy in enzymatic function. However, when testing the homologues for detoxification of trichothecenes, it was found that yeast expressing the gene with the highest sequence similarity UGT73C6 was as sensitive as wild-type to all toxins tested (as well as UGT73C3), while the second closest homologue UGT73C4 exhibited the same properties as DOGT1. Enzymatic activity towards different hydroxycoumarins has been shown for all 73C cluster members (Lim et al., Glycobiol. 13 (2003), 139-145), indicating that the UGTs lacking DON-protective activity are not simply loss of function alleles. The cluster is therefore an example that supports the hypothesis that duplicated UGT genes can acquire new substrate specificities and functions during evolution.

Plant UDP-glucosyltransferases are structurally very similar in their carboxy-terminal signature motif to mammalian UGTs which use UDP-glucuronic acid instead of UDP-glucose as donor substrate. The mammalian enzymes play a central role in metabolism and detoxification of chemicals like carcinogens or hydrophobic drugs. Higher plant UGTs have been found to be involved in a parallel range of activities also modifying xenobiotic substances such as herbicides and other pesticides. One open question is whether these detoxification reactions are side-activities of UGTs conjugating endogenous plant compounds. Although recent publications are in favor of the hypothesis that UGTs responsible for the conversion of endogenous substrates may also account for the capacity of plants to detoxify xenobiotic substances.

The construction of hybrid genes by shuffling the N- and C-terminal part of DOGT1 and UGT73C6 and their expression in yeast proved that the N-terminal 189 amino acids of DOGT1 are essential for its ability to detoxify DON, in agreement with the hypothesis that substrate binding may occur in a conserved hydrophobic area between amino acid 130 and 150 (Vogt et al. (1999), Plant J. 19/5:509-519). Comparison of two genes with protective effect and two genes without activity, but similar expression level, suggested that a lysine, which is present at amino acid position 136 in DOGT1 (and at the corresponding position in UGT73C4) could be important for specificity of these enzymes.

**Regulation of gene expression.** Genes with high sequence similarities to DOGT1 from tobacco and tomato have been shown to be induced by salicylic acid or wounding. The analysis of expression of DOGT1 and 73C6 following SA and JA treatment showed that they responded weekly with elevated mRNA levels to SA, JA and the ethylene precursor ACC. Inducibility of gene expression by SA, JA or ethylene is considered to be indicative for a possible role of the upregulated gene product in plant stress or defense responses.

With the present invention it was shown that the huge gene family of glycosyltransferases, especially glucosyltransferases, specifically - as shown in the examples - UGTs plays an important role in plant growth regulation.

## Claims

1. A method for regulating plant growth **characterised in that** the activity of a brassinosteroid specific glycosyltransferase is influenced.

2. A method according to claim 1, **characterised in that** plant growth is reduced by enhancing the expression of the brassinosteroid specific glycosyltransferase.

3. A method according to claim 1 or 2, **characterised in that** the method comprises functionally introducing in trans into a plant a heterologous glycosyltransferase and/or a heterologous expression regulating element for a glycosyltransferase.

4. A method according to any one of claims 1 to 3, **characterised in that** the brassinosteroid specific glycosyltransferase is a brassinosteroid specific glucosyltransferase, preferably an UDP-glucosyltransferase corresponding to subfamily 73C of Arabidopsis thaliana, especially UDP-glucosyltransferase 73C6, 73C5 and 73C4.

5. A method according to any one of claims 1 to 4, **characterised in that** the method comprises introducing a tissue specific promoter for the brassinosteroid specific glycosyltransferase, especially a stem specific promoter, into a plant.

6. A method according to any one of claims 1 to 5, **characterised in that** the plant is selected from the group containing Arabidopsis, rice, barley, wheat, tobacco, maize, sorghum, tomato, sun flower, fruit trees, ornamental plants, forest trees and agricultural plants, especially flowery plants, bonsai shrubs.

7. A method according to any one of claims 1 to 6, **characterised in that** the brassinosteroid specific glycosyltransferase is a glycosyltransferase being specific for campesterol, campestanol, brassinolide, stigmasterol, teasterone, methyldolichosterone, epibrassinolide, epicastasterone.

8. A method according to any one of claims 1 to 7, **characterised in that** plant growth is reduced by glucosylation of the C₂-OH, C₃-OH, C₂₃-OH, C₂₅-OH, C₂₆-OH and/or C₂₇-OH of brassinosteroids by brassinosteroid specific glucosyltransferases.

9. A method according to any one of claims 1 to 8 **characterised in that** the method comprises introducing an inducible promoter for the brassinosteroid specific glycosyltransferase, preferable a tissue specific promoter, especially a stem specific promoter, into a plant.

10. A recombinant cell comprising a heterologous glucosyltransferase or an enhanced expression activity of an endogenous glucosyltransferase due to transgenic expression regulating elements.

11. A cell according to claim 10, **characterised in that** it is a plant cell or a yeast cell.

12. A cell according to claim 10 or 11, **characterised in that** it comprises a tissue specific, especially a stem specific promoter.

13. Use of a heterologous glycosyltransferase for the production of a plant cell for regulating plant growth, especially for the production of a plant cell with reduced growth.

14. Method for producing glycosylated brassinosteroids **characterised in that** a brassinosteroid is contacted in vivo or in vitro by a glycosyltransferase in the presence of an activated glucose.


SEQUENCE LISTING

<110> Universität für Bodenkultur

<120> A method for regulating plant growth

<130> R 42960

<140> EP 04450028.8
<141> 2004-02-13

<160> 39

<170> PatentIn version 3.1

<210> 1
<211> 30
<212> DNA
<213> Artificial

<220>
<223> Description of Artificial Sequence:DNA

<400> 1
actaagcttg gaatcatggt ttccgaaaca 30

<210> 2
<211> 25
<212> DNA
<213> Artificial

<220>
<223> Description of Artificial Sequence:DNA

<400> 2
aagcggccgc atactcaatt attgg 25

<210> 3
<211> 31
<212> DNA
<213> Artificial

<220>
<223> Description of Artificial Sequence:DNA

<400> 3
ctaagcttgg aatcatggca tcggaatttc g 31

<210> 4
<211> 30
<212> DNA
<213> Artificial

<220>
<223>  Description of Artificial Sequence:DNA

<400>  4
tagcggccgc attcatttct tgggttgttc                                        30


<210>  5
<211>  32
<212>  DNA
<213>  Artificial

<220>
<223>  Description of Artificial Sequence:DNA

<400>  5
ctaagcttgg aatcatggct ttcgagaaga cc                                     32


<210>  6
<211>  30
<212>  DNA
<213>  Artificial

<220>
<223>  Description of Artificial Sequence:DNA

<400>  6
tagcggccgc attcaactct tggattctac                                        30


<210>  7
<211>  32
<212>  DNA
<213>  Artificial

<220>
<223>  Description of Artificial Sequence:DNA

<400>  7
ctaagcttgg aatcatggct acggaaaaaa cc                                     32


<210>  8
<211>  29
<212>  DNA
<213>  Artificial

<220>
<223>  Description of Artificial Sequence:DNA

<400>  8
tagcggccgc attcattctt gaattgtgc                                         29


<210>  9

<211>  31
<212>  DNA
<213>  Artificial

<220>
<223>  Description of Artificial Sequence:DNA

<400>  9
ctaagcttgg aatcatggct tccgaaaaat c                                    31


<210>  10
<211>  29
<212>  DNA
<213>  Artificial

<220>
<223>  Description of Artificial Sequence:DNA

<400>  10
tagcggccgc attcagttct tggatttca                                       29


<210>  11
<211>  31
<212>  DNA
<213>  Artificial

<220>
<223>  Description of Artificial Sequence:DNA

<400>  11
ctaagcttgg aacatgtgtt ctcatgatcc t                                    31


<210>  12
<211>  30
<212>  DNA
<213>  Artificial

<220>
<223>  Description of Artificial Sequence:DNA

<400>  12
tagcggccgc attcaattat tggactgtgc                                      30


<210>  13
<211>  59
<212>  DNA
<213>  Artificial

<220>
<223>  Description of Artificial Sequence:DNA

<400>  13

ggatgcccga acaaaagtta atttcagaag aggacttatc aaagcttgag gcctcgcga     59

<210>  14
<211>  26
<212>  DNA
<213>  Artificial

<220>
<223>  Description of Artificial Sequence:DNA

<400>  14
tacaagcgaa atcgccaaga agttca                                          26

<210>  15
<211>  26
<212>  DNA
<213>  Artificial

<220>
<223>  Description of Artificial Sequence:DNA

<400>  15
cttcttggcg atttcgcttg tataag                                          26

<210>  16
<211>  30
<212>  DNA
<213>  Artificial

<220>
<223>  Description of Artificial Sequence:DNA

<400>  16
actaagcttg gaatcatggt ttccgaaaca                                      30

<210>  17
<211>  25
<212>  DNA
<213>  Artificial

<220>
<223>  Description of Artificial Sequence:DNA

<400>  17
tcttgtgaat tcaactctat cagga                                           25

<210>  18
<211>  27
<212>  DNA
<213>  Artificial

<220>
<223>  Description of Artificial Sequence:DNA

<400>  18
tacaagcaaa atcgccaaga agttcaa                                          27


<210>  19
<211>  25
<212>  DNA
<213>  Artificial

<220>
<223>  Description of Artificial Sequence:DNA

<400>  19
acttcttggc gattttgctt gtatt                                            25


<210>  20
<211>  31
<212>  DNA
<213>  Artificial

<220>
<223>  Description of Artificial Sequence:DNA

<400>  20
taagcttgga atcatgtgtt ctcatgatcc t                                     31


<210>  21
<211>  26
<212>  DNA
<213>  Artificial

<220>
<223>  Description of Artificial Sequence:DNA

<400>  21
tcacccggga aacagtaatc atgtcc                                           26


<210>  22
<211>  22
<212>  DNA
<213>  Artificial

<220>
<223>  Description of Artificial Sequence:DNA

<400>  22
cgaggcagat cgtcagtcag tc                                               22


<210>  23

<211>  19
<212>  DNA
<213>  Artificial

<220>
<223>  Description of Artificial Sequence:DNA

<400>  23
atccggggtt gaacagcct                                                    19


<210>  24
<211>  19
<212>  DNA
<213>  Artificial

<220>
<223>  Description of Artificial Sequence:DNA

<400>  24
tcaattattg ggttctgcc                                                    19


<210>  25
<211>  19
<212>  DNA
<213>  Artificial

<220>
<223>  Description of Artificial Sequence:DNA

<400>  25
ggagaaaata ggagtgtta                                                    19


<210>  26
<211>  19
<212>  DNA
<213>  Artificial

<220>
<223>  Description of Artificial Sequence:DNA

<400>  26
tcagttcttg gatttcact                                                    19


<210>  27
<211>  18
<212>  DNA
<213>  Artificial

<220>
<223>  Description of Artificial Sequence:DNA

<400>  27

gagaaactgg tcgtacaa                                                    18

<210>  28
<211>  19
<212>  DNA
<213>  Artificial

<220>
<223>  Description of Artificial Sequence:DNA

<400>  28
tcaattattg gactgtgct                                                   19

<210>  29
<211>  21
<212>  DNA
<213>  Artificial

<220>
<223>  Description of Artificial Sequence:DNA

<400>  29
gtccttcttt ctggtaaacg t                                                21

<210>  30
<211>  21
<212>  DNA
<213>  Artificial

<220>
<223>  Description of Artificial Sequence:DNA

<400>  30
aacccttgag gttgaatcat c                                                21

<210>  31
<211>  38
<212>  DNA
<213>  Artificial

<220>
<223>  Description of Artificial Sequence:DNA

<400>  31
gttaaaagct tacatgtgca ttacggtctg tgtgaata                              38

<210>  32
<211>  38
<212>  DNA
<213>  Artificial

<220>
<223>  Description of Artificial Sequence:DNA

<400>  32
tttcggatcc catgattcaa ccttagtaag aaactctc                              38


<210>  33
<211>  495
<212>  PRT
<213>  Arabidopsis thaliana

<400>  33

Met Val Ser Glu Thr Thr Lys Ser Ser Pro Leu His Phe Val Leu Phe
1               5                   10                  15

Pro Phe Met Ala Gln Gly His Met Ile Pro Met Val Asp Ile Ala Arg
            20                  25                  30

Leu Leu Ala Gln Arg Gly Val Ile Ile Thr Ile Val Thr Thr Pro His
        35                  40                  45

Asn Ala Ala Arg Phe Lys Asn Val Leu Asn Arg Ala Ile Glu Ser Gly
    50                  55                  60

Leu Pro Ile Asn Leu Val Gln Val Lys Phe Pro Tyr Leu Glu Ala Gly
65                  70                  75                  80

Leu Gln Glu Gly Gln Glu Asn Ile Asp Ser Leu Asp Thr Met Glu Arg
                85                  90                  95

Met Ile Pro Phe Phe Lys Ala Val Asn Phe Leu Glu Glu Pro Val Gln
            100                 105                 110

Lys Leu Ile Glu Glu Met Asn Pro Arg Pro Ser Cys Leu Ile Ser Asp
        115                 120                 125

Phe Cys Leu Pro Tyr Thr Ser Lys Ile Ala Lys Lys Phe Asn Ile Pro
    130                 135                 140

Lys Ile Leu Phe His Gly Met Gly Cys Phe Cys Leu Leu Cys Met His
145                 150                 155                 160

Val Leu Arg Lys Asn Arg Glu Ile Leu Asp Asn Leu Lys Ser Asp Lys
                165                 170                 175

Glu Leu Phe Thr Val Pro Asp Phe Pro Asp Arg Val Glu Phe Thr Arg
            180                 185                 190

Thr Gln Val Pro Val Glu Thr Tyr Val Pro Ala Gly Asp Trp Lys Asp
        195                 200                 205

Ile Phe Asp Gly Met Val Glu Ala Asn Glu Thr Ser Tyr Gly Val Ile
    210                 215                 220

Val Asn Ser Phe Gln Glu Leu Glu Pro Ala Tyr Ala Lys Asp Tyr Lys
225                 230                 235                 240

Glu Val Arg Ser Gly Lys Ala Trp Thr Ile Gly Pro Val Ser Leu Cys
                245                 250                 255

Asn Lys Val Gly Ala Asp Lys Ala Glu Arg Gly Asn Lys Ser Asp Ile
            260                 265                 270

Asp Gln Asp Glu Cys Leu Lys Trp Leu Asp Ser Lys Lys His Gly Ser
        275                 280                 285

Val Leu Tyr Val Cys Leu Gly Ser Ile Cys Asn Leu Pro Leu Ser Gln
    290                 295                 300

Leu Lys Glu Leu Gly Leu Gly Leu Glu Glu Ser Gln Arg Pro Phe Ile
305                 310                 315                 320

Trp Val Ile Arg Gly Trp Glu Lys Tyr Lys Glu Leu Val Glu Trp Phe
                325                 330                 335

Ser Glu Ser Gly Phe Glu Asp Arg Ile Gln Asp Arg Gly Leu Leu Ile
            340                 345                 350

Lys Gly Trp Ser Pro Gln Met Leu Ile Leu Ser His Pro Ser Val Gly
        355                 360                 365

Gly Phe Leu Thr His Cys Gly Trp Asn Ser Thr Leu Glu Gly Ile Thr
    370                 375                 380

Ala Gly Leu Pro Leu Leu Thr Trp Pro Leu Phe Ala Asp Gln Phe Cys
385                 390                 395                 400

Asn Glu Lys Leu Val Val Glu Val Leu Lys Ala Gly Val Arg Ser Gly
                405                 410                 415

Val Glu Gln Pro Met Lys Trp Gly Glu Glu Glu Lys Ile Gly Val Leu
            420                 425                 430

Val Asp Lys Glu Gly Val Lys Lys Ala Val Glu Glu Leu Met Gly Glu
        435                 440                 445

Ser Asp Asp Ala Lys Glu Arg Arg Arg Arg Ala Lys Glu Leu Gly Asp
    450                 455                 460

Ser Ala His Lys Ala Val Glu Glu Gly Gly Ser Ser His Ser Asn Ile
465                 470                 475                 480

Ser Phe Leu Leu Gln Asp Ile Met Glu Leu Ala Glu Pro Asn Asn
                485                 490                 495

<210>  34
<211>  495
<212>  PRT
<213>  Vigna angularis

<400>  34

Met Val Leu Gln Thr Asn Asn Val Pro His Phe Val Leu Phe Pro Met
1               5                   10                  15

Met Ala Gln Gly His Ile Ile Pro Met Met Asp Ile Ala Arg Ile Leu
            20                  25                  30

Ala Gln Arg Gly Val Ile Ile Thr Val Phe Thr Thr Pro Lys Asn Ala
        35                  40                  45

Ser Arg Phe Asn Ser Val Ile Ser Arg Ala Val Ser Ser Gly Leu Lys
    50                  55                  60

Ile Arg Leu Val Gln Leu Asn Phe Pro Ser Lys Glu Ala Gly Leu Arg
65                  70                  75                  80

Glu Gly Cys Glu Asn Leu Asp Met Val Ser Ser Asn Asp Met Ser Lys
                85                  90                  95

Ile Phe Gln Val Ile His Met Pro Gln Lys Pro Ala Glu Glu Phe Phe
            100                 105                 110

Gln Thr Leu Thr Pro Lys Pro Ser Cys Ile Ile Ser Asp Phe Cys Ile
        115                 120                 125

Ala Trp Thr Leu Gln Leu Ala Glu Lys Tyr His Ile Pro Arg Val Ser
    130                 135                 140

Phe His Gly Phe Ser Cys Phe Cys Leu His Cys Arg Tyr Val Ile His
145                 150                 155                 160

Thr Ser Asp Phe Cys Arg Ser Ile Thr Ser Glu Ser Lys Tyr Phe Thr
                165                 170                 175

Ile Pro Gly Ile Pro Asp Lys Ile Gln Val Thr Lys Glu Gln Leu Pro
            180                 185                 190

Gly Ser Leu Ala Thr Asp Leu Asp Asp Phe Lys Asp Gln Val Arg Asp
        195                 200                 205

Ala Glu Lys Lys Ser Tyr Gly Val Ile Val Asn Thr Phe Trp Arg Val
    210                 215                 220

Gly Glu Gly Ile Cys Glu Gly Phe Ser Arg Arg Leu Lys Asn Asn Lys
225                 230                 235                 240

Ala Trp Phe Ile Gly Pro Val Ser Leu Cys Asn Lys Asp Gly Leu Asp
                245                 250                 255

Lys Ala Gln Arg Gly Lys Gln Ala Ser Ile Asn Glu Asn His Cys Leu
            260                 265                 270

Lys Trp Leu Asp Val Gln Gln Ala Lys Ser Val Val Tyr Val Cys Phe
        275                 280                 285

Gly Ser Ile Cys Asn Leu Ile Pro Ser Gln Leu Val Glu Leu Ala Leu
    290                 295                 300

Ala Leu Glu Asp Thr Lys Arg Pro Phe Val Trp Val Ile Arg Glu Gly
305                 310                 315                 320

Ser Gln Leu Gln Glu Leu Glu Lys Trp Phe Ser Glu Glu Gly Phe Glu
                325                 330                 335

Glu Arg Thr Lys Gly Arg Gly Leu Ile Ile Gly Gly Trp Ala Pro Gln
            340                 345                 350

Val Met Ile Leu Ser His Pro Ser Ile Gly Gly Phe Leu Thr His Cys
        355                 360                 365

Gly Trp Asn Ser Thr Leu Glu Gly Ile Cys Ala Gly Val Pro Leu Val
    370                 375                 380

Thr Trp Pro Leu Phe Gly Asp Gln Phe Leu Asn Glu Lys Pro Val Ser
385                 390                 395                 400

Asp Val Leu Arg Ile Gly Val Ser Val Gly Ala Glu Val Pro Leu Lys
                405                 410                 415

Trp Gly Glu Glu Glu Lys Arg Gly Val Met Val Lys Lys Asp Asp Ile
            420                 425                 430

Lys Arg Ala Ile Cys Met Val Met Asp Asp Glu Glu Gly Lys Glu Arg
        435                 440                 445

Arg Glu Arg Val Cys Lys Leu Ser Glu Met Gly Lys Arg Ala Val Glu
    450                 455                 460

Glu Gly Gly Ser Ser His Leu Asp Val Thr Leu Leu Ile Gln Asp Ile
465                 470                 475                 480

Met Gln Gln Thr Asn Thr Lys Glu Asp Glu Ser Leu Ser Asp Val
                485                 490                 495

<210>  35
<211>  476
<212>  PRT
<213>  Nicotiana tabacum

<400>  35

Met Gly Gln Leu His Phe Phe Phe Phe Pro Val Met Ala His Gly His
1               5                   10                  15

Met Ile Pro Thr Leu Asp Met Ala Lys Leu Phe Ala Ser Arg Gly Val
            20                  25                  30

Lys Ala Thr Ile Ile Thr Thr Pro Leu Asn Glu Phe Val Phe Ser Lys
        35                  40                  45

Ala Ile Gln Arg Asn Lys His Leu Gly Ile Glu Ile Glu Ile Arg Leu
    50                  55                  60

Ile Lys Phe Pro Ala Val Glu Asn Gly Leu Pro Glu Glu Cys Glu Arg
65                  70                  75                  80

Leu Asp Gln Ile Pro Ser Asp Glu Lys Leu Pro Asn Phe Phe Lys Ala
                85                  90                  95

Val Ala Met Met Gln Glu Pro Leu Glu Gln Leu Ile Glu Glu Cys Arg
            100                 105                 110

Pro Asp Cys Leu Ile Ser Asp Met Phe Leu Pro Trp Thr Thr Asp Thr
        115                 120                 125

Ala Ala Lys Phe Asn Ile Pro Arg Ile Val Phe His Gly Thr Ser Phe
    130                 135                 140

Phe Ala Leu Cys Val Glu Asn Ser Val Arg Leu Asn Lys Pro Phe Lys
145                 150                 155                 160

Asn Val Ser Ser Asp Ser Glu Thr Phe Val Val Pro Asp Leu Pro His
                165                 170                 175

Glu Ile Lys Leu Thr Arg Thr Gln Val Ser Pro Phe Glu Arg Ser Gly
            180                 185                 190

Glu Glu Thr Ala Met Thr Arg Met Ile Lys Thr Val Arg Glu Ser Asp
        195                 200                 205

Ser Lys Ser Tyr Gly Val Val Phe Asn Ser Phe Tyr Glu Leu Glu Thr
    210                 215                 220

Asp Tyr Val Glu His Tyr Thr Lys Val Leu Gly Arg Arg Ala Trp Ala
225                 230                 235                 240

Ile Gly Pro Leu Ser Met Cys Asn Arg Asp Ile Glu Asp Lys Ala Glu
                245                 250                 255

Arg Gly Lys Lys Ser Ser Ile Asp Lys His Glu Cys Leu Lys Trp Leu
            260                 265                 270

Asp Ser Lys Lys Pro Ser Ser Val Val Tyr Val Cys Phe Gly Ser Val
        275                 280                 285

Ala Asn Phe Thr Ala Ser Gln Leu His Glu Leu Ala Met Gly Ile Glu
    290                 295                 300

Ala Ser Gly Gln Glu Phe Ile Trp Val Val Arg Thr Glu Leu Asp Asn
305                 310                 315                 320

Glu Asp Trp Leu Pro Glu Gly Phe Glu Glu Arg Thr Lys Glu Lys Gly
                325                 330                 335

Leu Ile Ile Arg Gly Trp Ala Pro Gln Val Leu Ile Leu Asp His Glu
            340                 345                 350

Ser Val Gly Ala Phe Val Thr His Cys Gly Trp Asn Ser Thr Leu Glu
        355                 360                 365

Gly Val Ser Gly Gly Val Pro Met Val Thr Trp Pro Val Phe Ala Glu
    370                 375                 380

Gln Phe Phe Asn Glu Lys Leu Val Thr Glu Val Leu Lys Thr Gly Ala
385                 390                 395                 400

Gly Val Gly Ser Ile Gln Trp Lys Arg Ser Ala Ser Glu Gly Val Lys
                405                 410                 415

Arg Glu Ala Ile Ala Lys Ala Ile Lys Arg Val Met Val Ser Glu Glu
            420                 425                 430

Ala Asp Gly Phe Arg Asn Arg Ala Lys Ala Tyr Lys Glu Met Ala Arg
        435                 440                 445

Lys Ala Ile Glu Glu Gly Gly Ser Ser Tyr Thr Gly Leu Thr Thr Leu
    450                 455                 460

Leu Glu Asp Ile Ser Thr Tyr Ser Ser Thr Gly His
465                 470                 475

<210>  36
<211>  476
<212>  PRT
<213>  Nicotiana tabacum

<400>  36

Met Gly Gln Leu His Ile Phe Phe Phe Pro Val Met Ala His Gly His
1               5                   10                  15

Met Ile Pro Thr Leu Asp Met Ala Lys Leu Phe Ala Ser Arg Gly Val
            20                  25                  30

Lys Ala Thr Ile Ile Thr Thr Pro Leu Asn Glu Phe Val Phe Ser Lys
        35                  40                  45

Ala Ile Gln Arg Asn Lys His Leu Gly Ile Glu Ile Glu Ile Arg Leu
    50                  55                  60

Ile Lys Phe Pro Ala Val Glu Asn Gly Leu Pro Glu Glu Cys Glu Arg
65                  70                  75                  80

Leu Asp Gln Ile Pro Ser Asp Glu Lys Leu Pro Asn Phe Phe Lys Ala
                85                  90                  95

Val Ala Met Met Gln Glu Pro Leu Glu Gln Leu Ile Glu Glu Cys Arg
            100                 105                 110

Pro Asp Cys Leu Ile Ser Asp Met Phe Leu Pro Trp Thr Thr Asp Thr
        115                 120                 125

Ala Ala Lys Phe Asn Ile Pro Arg Ile Val Phe His Gly Thr Ser Phe
    130                 135                 140

Phe Ala Leu Cys Val Glu Asn Ser Val Arg Leu Asn Lys Pro Phe Lys
145                 150                 155                 160

Asn Val Ser Ser Asp Ser Glu Thr Phe Val Val Pro Asp Leu Pro His
                165                 170                 175

Glu Ile Lys Leu Thr Arg Thr Gln Val Ser Pro Phe Glu Arg Ser Gly

180                 185                 190

Glu Glu Thr Ala Met Thr Arg Met Ile Lys Thr Val Arg Glu Ser Asp
        195                 200                 205

Ser Lys Ser Tyr Gly Val Val Phe Asn Ser Phe Tyr Glu Leu Glu Thr
    210                 215                 220

Asp Tyr Val Glu His Tyr Thr Lys Val Leu Gly Arg Arg Ala Trp Ala
225                 230                 235                 240

Ile Gly Pro Leu Ser Met Cys Asn Arg Asp Ile Glu Asp Lys Ala Glu
                245                 250                 255

Arg Gly Lys Lys Ser Ser Ile Asp Lys His Glu Cys Leu Lys Trp Leu
            260                 265                 270

Asp Ser Lys Lys Pro Ser Ser Val Val Tyr Ile Cys Phe Gly Ser Val
        275                 280                 285

Ala Asn Phe Thr Ala Ser Gln Leu His Glu Leu Ala Met Gly Val Glu
    290                 295                 300

Ala Ser Gly Gln Glu Phe Ile Trp Val Val Arg Thr Glu Leu Asp Asn
305                 310                 315                 320

Glu Asp Trp Leu Pro Glu Gly Phe Glu Glu Arg Thr Lys Glu Lys Gly
                325                 330                 335

Leu Ile Ile Arg Gly Trp Ala Pro Gln Val Leu Ile Leu Asp His Glu
            340                 345                 350

Ser Val Gly Ala Phe Val Thr His Cys Gly Trp Asn Ser Thr Leu Glu
        355                 360                 365

Gly Val Ser Gly Gly Val Pro Met Val Thr Trp Pro Val Phe Ala Glu
    370                 375                 380

Gln Phe Phe Asn Glu Lys Leu Val Thr Glu Val Leu Lys Thr Gly Ala
385                 390                 395                 400

Gly Val Gly Ser Ile Gln Trp Lys Arg Ser Ala Ser Glu Gly Val Lys

                405                 410                 415

Arg Glu Ala Ile Ala Lys Ala Ile Lys Arg Val Met Val Ser Glu Glu
            420                 425                 430

Ala Asp Gly Phe Arg Asn Arg Ala Lys Ala Tyr Lys Glu Met Ala Arg
        435                 440                 445

Lys Ala Ile Glu Glu Gly Gly Ser Ser Tyr Thr Gly Leu Thr Thr Leu
    450                 455                 460

Leu Glu Asp Ile Ser Thr Tyr Ser Ser Thr Gly His
465                 470                 475

<210> 37
<211> 497
<212> PRT
<213> Oryza sativa

<400> 37

Met Ser Ser Ala Gly His Ala Val Asp Gln Gln Arg Lys Ser Thr Thr
1               5                   10                  15

Met Lys Ala His Phe Val Leu Val Pro Met Met Ala Gln Gly His Met
            20                  25                  30

Ile Pro Met Thr Gly Met Ala Arg Leu Leu Ala Glu His Gly Ala Gln
        35                  40                  45

Val Ser Phe Val Thr Thr Pro Val Asn Ala Ala Arg Met Ala Gly Phe
    50                  55                  60

Val Thr Ala Val Glu Ala Ala Gly Leu Ala Val Gln Leu Val Lys Leu
65                  70                  75                  80

Pro Phe Pro Ala Thr Glu Phe Gly Leu Pro Asp Gly Cys Glu Asn Leu
                85                  90                  95

Asp Met Ile Gln Ser Arg Asp Leu Ser Arg Asn Phe Met Glu Ala Cys
            100                 105                 110

Gly Ala Leu Arg Glu Pro Leu Thr Ala Arg Leu Arg Gln Leu Cys Pro
        115                 120                 125

Pro Pro Ser Cys Ile Ile Ser Asp Met Val Gln Trp Trp Thr Gly Glu
    130                 135                 140

Ile Ala Arg Glu Leu Gly Ile Pro Arg Leu Thr Phe Asp Gly Phe Cys
145                 150                 155                 160

Thr Phe Ala Ser Leu Ala Arg Tyr Ile Ile Phe Arg Asp Lys Leu Leu
                165                 170                 175

Asp Asn Val Ala Asp Glu Glu Ile Val Thr Phe Ser Gly Phe Pro Met
            180                 185                 190

Leu Leu Glu Leu Pro Lys Ala Arg Cys Pro Gly Ser Leu Cys Val Pro
        195                 200                 205

Gly Met Glu Gln Ile Arg Asp Lys Met Tyr Glu Glu Glu Leu Gln Ser
    210                 215                 220

Asp Gly Asn Val Met Asn Ser Phe Gln Glu Leu Glu Thr Leu Tyr Ile
225                 230                 235                 240

Glu Ser Phe Glu Gln Ile Thr Gly Lys Lys Val Trp Thr Ile Gly Pro
                245                 250                 255

Met Cys Leu Cys Asp Arg Asp Ser Asn Met Met Ala Ala Arg Gly Asn
            260                 265                 270

Lys Ala Ser Val Asp Glu Ala Lys Cys Leu Gln Trp Leu Asp Ser Lys
        275                 280                 285

Lys Pro Gly Ser Val Ile Phe Val Ser Phe Gly Ser Leu Ala Ser Thr
    290                 295                 300

Ala Pro Gln Gln Leu Val Glu Leu Gly Leu Gly Leu Glu Ala Ser Lys
305                 310                 315                 320

Glu Pro Phe Ile Trp Val Ile Lys Ala Gly Asn Lys Phe Pro Glu Val
                325                 330                 335

Glu Glu Trp Leu Ala Asp Gly Phe Glu Glu Arg Val Lys Asp Arg Gly
            340                 345                 350

Met Ile Ile Arg Gly Trp Ala Pro Gln Val Met Ile Leu Trp His Gln
        355                 360                 365

Ala Ile Gly Gly Phe Met Thr His Cys Gly Trp Asn Ser Thr Ile Glu
    370                 375                 380

Gly Ile Cys Ala Gly Val Pro Met Ile Thr Trp Pro His Phe Ala Glu
385                 390                 395                 400

Gln Phe Leu Asn Glu Lys Phe Val Val Asn Leu Leu Lys Ile Gly Leu
                405                 410                 415

Glu Ile Gly Val Lys Gly Val Ala Gln Trp Gly Ser Glu His Lys Glu
            420                 425                 430

Val Arg Val Thr Arg Asn Ala Val Glu Thr Ala Val Ser Thr Leu Met
        435                 440                 445

Asn Asp Gly Glu Ala Ala Gln Glu Met Arg Met Arg Ala Lys Asp Leu
    450                 455                 460

Gly Val Lys Ala Arg Arg Ala Leu Glu Glu Gly Gly Ser Ser Tyr Asp
465                 470                 475                 480

Asn Ile Ser Leu Leu Ile Gln Glu Met Gly Asn Lys Gln Asn Ala Ser
                485                 490                 495

Gly

<210>  38
<211>  466
<212>  PRT
<213>  Lycopersicon esculentum

<400>  38

His Phe Phe Phe Phe Pro Asp Asp Ala Gln Gly His Met Ile Pro Thr
1               5                   10                  15

Leu Asp Met Ala Asn Val Val Ala Cys Arg Gly Val Lys Ala Thr Ile
            20                  25                  30

Ile Thr Thr Pro Leu Asn Glu Ser Val Phe Ser Lys Ala Ile Glu Arg
        35                  40                  45

Asn Lys His Leu Gly Ile Glu Ile Asp Ile Arg Leu Leu Lys Phe Pro
    50                  55                  60

Ala Lys Glu Asn Asp Leu Pro Glu Asp Cys Glu Arg Leu Asp Leu Val
65                  70                  75                  80

Pro Ser Asp Asp Lys Leu Pro Asn Phe Leu Lys Ala Ala Ala Met Met
                85                  90                  95

Lys Asp Glu Phe Glu Glu Leu Ile Gly Glu Cys Arg Pro Asp Cys Leu
            100                 105                 110

Val Ser Asp Met Phe Leu Pro Trp Thr Thr Asp Ser Ala Ala Lys Phe
        115                 120                 125

Ser Ile Pro Arg Ile Val Phe His Gly Thr Ser Tyr Phe Ala Leu Cys
    130                 135                 140

Val Gly Asp Thr Ile Arg Arg Asn Lys Pro Phe Lys Asn Val Ser Ser
145                 150                 155                 160

Asp Thr Glu Thr Phe Val Val Pro Asp Leu Pro His Glu Ile Arg Leu
                165                 170                 175

Thr Arg Thr Gln Leu Ser Pro Phe Glu Gln Ser Asp Glu Glu Thr Gly
            180                 185                 190

Met Ala Pro Met Ile Lys Ala Val Arg Glu Ser Asp Ala Lys Ser Tyr
        195                 200                 205

Gly Val Ile Phe Asn Ser Phe Tyr Glu Leu Glu Ser Asp Tyr Val Glu
    210                 215                 220

His Tyr Thr Lys Val Val Gly Arg Lys Asn Trp Ala Ile Gly Pro Leu
225                 230                 235                 240

Ser Leu Cys Asn Arg Asp Ile Glu Asp Lys Ala Glu Arg Gly Arg Lys
                245                 250                 255

Ser Ser Ile Asp Glu His Ala Cys Leu Lys Trp Leu Asp Ser Lys Lys
            260                 265                 270

Ser Ser Ser Ile Val Tyr Val Cys Phe Gly Ser Thr Ala Asp Phe Thr
        275                 280                 285

Thr Ala Gln Met Gln Glu Leu Ala Met Gly Leu Glu Ala Ser Gly Gln
    290                 295                 300

Asp Phe Ile Trp Val Ile Arg Thr Gly Asn Glu Asp Trp Leu Pro Glu
305                 310                 315                 320

Gly Phe Glu Glu Arg Thr Lys Glu Lys Gly Leu Ile Ile Arg Gly Trp
                325                 330                 335

Ala Pro Gln Ser Val Ile Leu Asp His Glu Ala Ile Gly Ala Phe Val
            340                 345                 350

Thr His Cys Gly Trp Asn Ser Thr Leu Glu Gly Ile Ser Ala Gly Val
        355                 360                 365

Pro Met Val Thr Trp Pro Val Phe Ala Glu Gln Phe Phe Asn Glu Lys
    370                 375                 380

Leu Val Thr Glu Val Met Arg Ser Gly Ala Gly Val Gly Ser Lys Gln
385                 390                 395                 400

Trp Lys Arg Thr Ala Ser Glu Gly Val Lys Arg Glu Ala Ile Ala Lys
                405                 410                 415

Ala Ile Lys Arg Val Met Ala Ser Glu Glu Thr Glu Gly Phe Arg Ser
            420                 425                 430

Arg Ala Lys Glu Tyr Lys Glu Met Ala Arg Glu Ala Ile Glu Glu Gly
        435                 440                 445

Gly Ser Ser Tyr Asn Gly Trp Ala Thr Leu Ile Gln Asp Ile Thr Ser
    450                 455                 460

Tyr Arg
465

<210>  39
<211>  489
<212>  PRT
<213>  Dorothenathus bellidiformis

<400>  39

Met Gly Thr His Ser Thr Ala Pro Asp Leu His Val Val Phe Phe Pro
1               5                   10                  15

Phe Leu Ala His Gly His Met Ile Pro Ser Leu Asp Ile Ala Lys Leu
            20                  25                  30

Phe Ala Ala Arg Gly Val Lys Thr Thr Ile Ile Thr Thr Pro Leu Asn
        35                  40                  45

Ala Ser Met Phe Thr Lys Ala Ile Glu Lys Thr Arg Lys Asn Thr Glu
    50                  55                  60

Thr Gln Met Glu Ile Glu Val Phe Ser Phe Pro Ser Glu Glu Ala Gly
65                  70                  75                  80

Leu Pro Leu Gly Cys Glu Asn Leu Glu Gln Ala Met Ala Ile Gly Ala
                85                  90                  95

Asn Asn Glu Phe Phe Asn Ala Ala Asn Leu Leu Lys Glu Gln Leu Glu
            100                 105                 110

Asn Phe Leu Val Lys Thr Arg Pro Asn Cys Leu Val Ala Asp Met Phe
        115                 120                 125

Phe Thr Trp Ala Ala Asp Ser Thr Ala Lys Phe Asn Ile Pro Thr Leu
    130                 135                 140

Val Phe His Gly Phe Ser Phe Phe Ala Gln Cys Ala Lys Glu Val Met
145                 150                 155                 160

Trp Arg Tyr Lys Pro Tyr Lys Ala Val Ser Ser Asp Thr Glu Val Phe
                165                 170                 175

Ser Leu Pro Phe Leu Pro His Glu Val Lys Met Thr Arg Leu Gln Val
            180                 185                 190

Pro Glu Ser Met Arg Lys Gly Glu Glu Thr His Phe Thr Lys Arg Thr
        195                 200                 205

Glu Arg Ile Arg Glu Leu Glu Arg Lys Ser Tyr Gly Val Ile Val Asn
    210                 215                 220

Ser Phe Tyr Glu Leu Glu Pro Asp Tyr Ala Asp Phe Leu Arg Lys Glu
225                 230                 235                 240

Leu Gly Arg Arg Ala Trp His Ile Gly Pro Val Ser Leu Cys Asn Arg
                245                 250                 255

Ser Ile Glu Asp Lys Ala Gln Arg Gly Arg Gln Thr Ser Ile Asp Glu
            260                 265                 270

Asp Glu Cys Leu Lys Trp Leu Asn Ser Lys Lys Pro Asp Ser Val Ile
        275                 280                 285

Tyr Ile Cys Phe Gly Ser Thr Gly His Leu Ile Ala Pro Gln Leu His
    290                 295                 300

Glu Ile Ala Thr Ala Leu Glu Ala Ser Gly Gln Asp Phe Ile Trp Ala
305                 310                 315                 320

Val Arg Gly Asp His Gly Gln Gly Asn Ser Glu Glu Trp Leu Pro Pro
                325                 330                 335

Gly Tyr Glu His Arg Leu Gln Gly Lys Gly Leu Ile Ile Arg Gly Trp
            340                 345                 350

Ala Pro Gln Val Leu Ile Leu Glu His Glu Ala Thr Gly Gly Phe Leu
        355                 360                 365

Thr His Cys Gly Trp Asn Ser Ala Leu Glu Gly Ile Ser Ala Gly Val
    370                 375                 380

Pro Met Val Thr Trp Pro Thr Phe Ala Glu Gln Phe His Asn Glu Gln
385                 390                 395                 400

Leu Leu Thr Gln Ile Leu Lys Val Gly Val Ala Val Gly Ser Lys Lys
                405                 410                 415

Trp Thr Leu Lys Pro Ser Ile Glu Asp Val Ile Lys Ala Glu Asp Ile
            420                 425                 430

Glu Lys Ala Val Arg Glu Val Met Val Gly Glu Glu Gly Glu Glu Arg
        435                 440                 445

Arg Arg Arg Ala Lys Lys Leu Lys Glu Met Ala Trp Arg Ala Ile Glu
    450                 455                 460

Glu Gly Gly Ser Ser Tyr Ser Asp Leu Ser Ala Leu Ile Glu Glu Leu
465                 470                 475                 480

Lys Gly Tyr His Thr Ser Glu Lys Glu
                485